# EUROPEAN PATENT SPECIFICATION

(11) **EP 3 460 077 B1**
(45) Date of publication and mention of the grant of the patent: **17.06.2020**
(21) Application number: 17192156.2
(22) Date of filing: 20.09.2017
(51) Int. Cl.: C12Q 1/6816, G01N 27/00, C12Q 1/6825

(54) **DIAGNOSTIC DEVICE AND SYSTEM**
DIAGNOSEVORRICHTUNG UND SYSTEM
DISPOSITIF ET SYSTÈME DE DIAGNOSTIC

(43) Date of publication of application: 27.03.2019
(73) Proprietor: Altratech Limited, Limerick, V94 AT85 (IE)
(72) Inventor: O'FARRELL, Brian, County Cork (IE); O'SULLIVAN, Cian Desmond, Limerick (IE); O'DRISCOLL, John, County Cork, (IE); CUMMINS, Timothy, County Clare, (IE); FREE, Paul, Haverhill, Suffolk CB9 9LP (GB); MCCARTHY, Moira, County Cork (IE)
(74) Representative: John A. O'Brien & Associates

(56) References cited:
- WO-A1-2015/086652
- WO-A2-2009/111316
- US-A1- 2015 118 743

## Description

### Introduction

The invention relates to human or animal medical diagnostics.

WO2015086652A1 describes preparing a nucleic acid sample involving lysing cells and using PNA probes to capture RNA in the cells.

WO2015091139A3 describes preparing a nucleic acid sample involving the step of tethering PNA-coated beads.

WO2015086654A1 describes capacitive sensors arranged to detect and quantify an analyte in a liquid sample over a sensor surface.

US2015118743 describes a nucleic acid extraction device having a tube extending in a longitudinal direction in which a first plug formed of a wax or an oil, a second plug formed of a first washing liquid, a third plug formed of a wax, a fourth plug formed of an eluate, and a fifth plug formed of a wax or an oil are arranged in this order.

The present invention is directed towards providing a device and system for testing a sample such as a blood sample and providing clear diagnostic data, in which:
the device is compact and portable, with few components; and/or
has the ability to detect and quantify single or multiple target nucleic acids, such as RNA (Ribonucleic Acid), or other microbiological entities, e.g. viral RNA from five or more species with replication and suitable controls; and/or
has the ability to assay multiple RNAs; and/or
the major parts of the device are fully synthetic (e.g. no enzymatic reagents) to allow for reproducibility, stability and simple logistics; and/or
is inexpensive enough to be disposable; and/or
the device reader avoids need for a service engineer.

### Summary of the Invention

In various aspects, we describe a diagnostic device which has stages linked by channels with cross-sectional areas preferably in the range of 0.02 mm² and 1.5 mm2, and more preferably in the range of 0.05mm² to 0.8 mm². The channel walls are preferably thin (such as less than 3mm) to allow for good thermal conductivity and penetration of magnetic fields. The channels and stages may be formed by a length of tubing in which the stages are defined by barriers, or the channels may be conduits linking physically discrete stages. Magnetic particles used for capturing and transporting the analyte (transport or "T beads") may be initially contained in a wax and bead matrix plug 104 which is solid at ambient temperature (4-50C). Where channel segments form stages they are preferably bounded by oil or other barrier liquid or gel which allows passage of analyte to the next stage. Cohesive forces between the water molecules exclude oil molecules (e.g. silicone oil or cycloalkanes) creating an aqueous phase/oil phase interface and barrier to the transition of hydrophilic molecules and hydrophilic particles. Entropy determines that the surface areas of the interfaces are kept to a minimum.

Magnetic particles ("T" beads) suspended in the aqueous phase (i.e. they have a hydrophilic surface) experience a force when an external force is applied, e.g. by a permanent magnet. As the particles are concentrated and are moved towards the aqueous/oil interface, this force is now exerted on the interface. Frictional forces on the bolus are minimised by keeping the beads away from the microfluidic channel wall using magnets and use of Teflon-coated microfluidics. An aqueous bolus forms around the magnetic particles as the magnetic force pulls them into the oil. Interfacial tension maintains the bolus of water (and any impurities therein) around the magnetic particles. Entropy determines that the surface area of this new aqueous/oil interface is kept to a minimum so this bolus is very small (100-500nl). This minimises the volume of liquid and the amount of impurities carried with the magnetic particles whereas all captured analyte is maintained on the surface of the particles. The analyte captured on the magnetic particles is concentrated in this bolus. They are also concentrated relative to any impurities.

Probes and/or transport beads may be microencapsulated (e.g. ethyl cellulose, polyvinyl alcohol, gelatin, sodium alginate) prior to being incorporated in a wax. This may provide for better stability and controlled release of the particles during the assay.

The device may be used by external application of heat either manually or in an automated manner in a reader, and likewise movement may be caused magnetically either manually or in an automated manner.

There may be a first wash stage in which "T beads" are actively mixed using external magnets to release any non-target biological material which may have been carried within the bolus through a wax barrier from a lysis stage.

There may be a reporter probe attachment stage for use after washing of the analyte has been completed. This may contain a PNA probe (Probe2) which is complementary to a different section of the target NA than the original Probe1. The reporter Probe2 may be covalently attached to a reporter, such as a bead ("R"-bead). These R-beads may be smaller in diameter and are less magnetically susceptible than the T-beads. T-beads may be mixed within this reservoir, and if any target NA is present on the T-beads they bind and become attached to PNA Probe2 on the R-beads. This binding of the two probes across the same NA results in the T-beads and R-beads being tethered together only if the NA target is present. The sandwich comprises: T-bead - Probe 1 - target NA - Probe2 - R-bead.

The T-beads may be once again magnetically aggregated and removed into a second wash stage at which they are once again mixed and separated into solution, this time to release any non-attached R-beads which may have been carried through from the previous capture phase. The T-beads may then be aggregated once again and pulled through the oil barrier 112 into a sensor. There is now a 1:1 ratio of R-beads to NA targets. This is because the R-beads can only get from one reservoir to another reservoir if tethered to the T-bead by the Probe1 - target NA - Probe2 sandwich.

The T-beads may be agitated over the sensor, such as by being magnetically moved over the surface of the sensor. The R-beads are separated from the T-beads and NA. This may be accomplished by application of heat to melt the PNA-Probe2 links to the NA. The magnetic T-beads can now be pulled away either in the same direction or alternatively back into the previous stage for a second time. This leaves the R-beads over the sensor surface.

The Probe2 heating temperature to separate the second probes is typically 60°C to 85°C, depending on its exact sequence which affects hybridisation strength. This temperature may be achieved by placing the device substrate on a hot plate, and/or or by using an induction coil , which enables magnetic induction heating of ferrite R-beads, as described in US5,378,879.

The presence of the isolated R-beads on the sensor indicates the presence of target NA in the original sample.

### Multiplexing

Multiple NA targets could be targeted in parallel using the above approach, e.g. for detecting two types of virus at the same time (HIV_T-bead - HIV_Probe1 - HIV_target NA - HIV_Probe2 - HIV_R-bead; HCV_T-bead - HCV_Probe1 - HCV_target NA - HCV_Probe2 - HCV_R-bead). This results in two types of R beads arriving on the sensor. The sensor can determine the quantities of each type of R bead. Alternatively, the T-bead could have both HIV_Probe1 and HCV_Probe1 but with separate HIV_R-bead and HCV_R-bead.

The sensor may have at least two sensor regions with PNA probes which are complementary to PNAs on the R beads so that each type of R bead becomes hybridised to the correct sensor.

The PNA probes on the sensor may be complementary to the HIV_Probe2 and HCV_Probe2.

Alternatively, the PNA probes on the sensor may be complementary to a standard PNA probe on each type of R bead. These R beads would then be additionally functionalised with HIV_Probe2 and HCV_Probe2. As the sensors come manufactured with standard PNAs, this approach allows more rapid assay development.

### Sensing Stage

The sensor may include a semiconductor capacitance sensor chip mounted in the encapsulated microfluidic structure having a high-resolution sigma-delta capacitance-to-digital converter, calibration memory, and digital processing circuitry, including I2C serial communication. Terminals may connect the chip to an external reader or computer. A first "wet-capacitance" reading is taken by the sensor chip, of the R-beads in liquid. This provides a baseline reference calibration capacitance. After the liquid evaporates, a second "dry capacitance" reading is taken of the R-bead capacitance. Due to their dielectric constant being higher than air, any R-beads on the sensor surface give a capacitive signal, e.g. IfF (delta capacitance versus dry air) for 200 beads, as shown in Fig. 19 of WO2015086654.

The PNA probes on the sensor may be complementary to a standard PNA probe on each type of R bead. These R beads would then be additionally functionalised with HIV_Probe2 or HCV_Probe2. As the sensors come manufactured with standard PNAs, this approach allows more rapid assay development.

The probe may also contain cell-penetrating peptides (e.g. trans-activating transcriptional activator TAT) to facilitate cellular uptake prior to lysis. This limits exposure of the NA target to nucleases prior to lysis. This would be particularly beneficial in the case of PNA probes wherein nucleases do not recognise the PNA-NA duplex. The probes could be bound to particles or biotinylated for capture by streptavidin-coated beads.

Sample treatment (e.g. a hot plate which can be adjusted to specific temperatures) so as to only cause lysis of certain types of cells or capsids in the sample. Such an adjust may in one example be applying a stress (in this case heat) to all cells within a blood sample wherein white blood cells (containing DNA) can respond to prevent lysis whereas other cells and, particularly, viral particles are lysed. This is advantageous where the white blood cells contain retroviral DNA which can confound an assay for retroviral RNA. This retroviral DNA will not be available within the lysate produced here.

A lysis buffer can be adjusted so as to only cause lysis in certain target cells or capsids in the sample. This adjustment may for example involve a drug designed to bind to a particular protein target on the viral surface which damages membrane integrity.

Interdigitated electrodes on the sensor may be arranged to act as a "snag trap" for R-beads. This may be accomplished by orienting them perpendicular to the flow of beads and controlling the pitch of the electrodes so that only the small R beads can fit between them.

Blood may pass through immunomagnetic beads suitable for removing certain fractions of the blood prior to application of the sample to the device (e.g. Whole Blood MicroBeads Miltenyi Biotec).

The blood may pass through a bed of resinous beads which may be functionalised (e.g. Chelex) or coated in reagents to help prevent clotting (other chelating agents such as EDTA or citrate).

These beads could also be functionalised with ligands for selective capture of certain fractions of the blood after application of the sample to the device (following same principle of Whole Blood MicroBeads Miltenyi Biotec).

These beads may be of a high density and gravity will hold these *in situ,* particularly where this section is vertical.

The reader may have a point heater on movable arms, for accurate heating control.

Where there are organic solvents, it will be clear that the lower density allows gravity to assist R-beads to drop across (vertical configuration) or down (horizontal configuration) on to the chip faster due to their lower buoyancy in the alcohol solution. This is desirable for some applications.

In one aspect, a portable diagnostic device comprises:
a series of assay stages , each with a reservoir linked by channels; and
at least one of the assay stages including a sensor or an interface to a sensor for detecting analyte which is optionally attached to beads.

The device may further comprise a lysate stage for receiving a sample and containing magnetic beads with a probe, and a lysate agent and an outlet to the assay stages.

Preferably, the lysate stage comprises a liquid-liquid purification stage in an outlet port, the purification stage comprising a wax plug including embedded magnetic beads for attachment to target analyte, providing for concentration and separation of the analyte from other material as it passes through the liquid barrier.

The lysate stage outlet port may be funnel-shaped and the wax plug is a friction fit within the outlet port.

The magnetic beads may be embedded in a wax plug comprising a composite of polystyrene and ferrite nanoparticles. Preferably, at least one assay stage is configured to carry out a wash. Preferably, said wash stage is arranged to provide interfacial tension and capillary forces between the water and oil/wax barriers, keeping the aqueous solution reservoirs isolated from each other.

Preferably, a single, cohesive oil and/or wax component is used as a barrier in non-sequential assay steps. Preferably, the wash stage comprises a reservoir with a buffer such as aqueous solution, the reservoir being arranged to allow mixing to facilitate beads to homogenise in the buffer.

Preferably, the wash assay stage is connected by a channel to a reporter bead (R-bead) assay stage. Preferably, the reporter bead assay stage contains magnetic reporter beads which have less magnetism than transport beads, and the reporter beads have attached a second PNA-probe ("Probe2") complementary to target nucleic acid of the analyte, such that if the target NA is present attached to a transport bead (T-bead), a T-bead-R-bead tethered sandwich is formed within the assay stage.

Preferably, the reporter beads have a size in the range of 0.1µm to 0.5µm.

The reporter bead assay stage may be connected by a channel to a reporter bead wash stage, for removing any excess R-beads. Preferably, the reporter bead wash assay stage is connected by a channel to a next assay stage which comprises the sensor.

Preferably, the sensor comprises a capacitive sensor arranged to detect capacitance of contents of an associated reservoir. The sensor may be adapted to perform detection based on capacitance to detect the number of reporter beads on the sensor surface, in which the reporter beads represent quantity of analyte such as target NA.

Preferably, the sensor is adapted to perform detection of a wet sample and/or to perform detection of a dry sample.

Preferably, the device further comprises an agitator for agitation of analyte over the sensor. Preferably, the agitator comprises a piston for pushing and pulling analyte over the sensor, and such a piston may be driven by a screw mechanism, and the piston may be arranged to push a reference fluid over the sensor, and the sensor may be a capacitive sensor and the piston is configured to push a gas with a known dielectric constant over the sensor.

Preferably, the sensor comprises a processor configured to store instructions and data required for controlling a reader and conducting the assay, optionally including instructions for actuator movements and temperature cycles.

Preferably, said processor is configured to drive the reader so that assays are conducted automatically on insertion of the device into the reader. The sensor may be arranged to read beads on its surface against a background of an organic liquid having a low dielectric constant.

Preferably, the sensor comprises PNA probes which are complementary to a standard PNA probe on each type of R bead, and the R beads are additionally functionalised with HIV_Probe2 and HCV_Probe2.

The sensor may comprise electrodes arranged to act as a trap for beads such as reporter beads. Preferably, the trap comprises electrodes arranged perpendicular to a flow of beads and controlling the pitch of the electrodes so that only the small R beads can fit between them.

Preferably, the device comprises a waste chamber with a vent region, into which displaced liquid such as molten wax; chip reservoir buffer; surfactant; and any gas, may be pushed. Preferably, the expansion chamber contains a volume of sterile gas which may be pushed in front of this liquid through a filtered vent to the environment.

The device may further comprise a heater for heating sample in the lysis stage and/or analyte in at least one assay stage. Preferably, the device channel comprises a heterogeneous thermally-conductive matrix, and optionally also includes a hydrophobic coating.

Preferably, the device is configured to fit into a reader to form a sensing system, and optionally the device includes a processor configured to communicate with the reader to provide assay instructions and data so that that an assay is controlled by the device. Preferably, at least some of the channel is exposed to allow magnetic movement of beads by a magnet, and optionally at least some of the channel is exposed to be heated by a heater, and preferably has a thin wall of less than 1mm for ease of heat transfer.

The device may include a processor configured to store and communicate a unique identifier for a sample, and optionally this identifier is correlated with a physical identifier printed on the device housing for reading by for example a 1D or 2D code reader.

We also describe a portable diagnostic system comprising a device of any embodiment and a reader, the reader comprising:
a support for receiving the device;
magnets on a drive to convey beads through the assay stages of the device;
a heat source;
a means of connection with the sensor of the device, or a sensor for coupling with the sensor interface of the device, and
a controller.

Preferably, the magnet drive comprises at least one arm arranged to move alongside the device. There may be at least two drive arms, arranged to move on opposed sides of the device. Preferably, the heat source is a thin foil heater and/or an overlay moulded to the device.

Preferably, the magnets include a conical magnet wherein magnetic field lines are concentrated at an apex of the magnet. The magnets may be arranged to have a shape such as conical and/or orientation to provide a steep gradient in magnetic field for at least one assay stage.

The magnets may be mounted to be separated by a distance in the range of 5 mm to 20 mm from the device. The magnets may have a drive to vary separation of the magnets from the device, for analyte mixing.

Preferably, the magnets have a drive for oscillating a magnet back and forth against another static magnet. Preferably, the magnets have a drive for rotating a magnet to create an oscillating magnetic field on one side of a channel.

The magnets may have a drive with a static magnet on one side and an oscillating magnet on the opposed side of a channel. The heater may comprise a point heater on moveable arms.

Preferably, the sensor is arranged to read beads on its surface against a background of an organic liquid such as ethanol.

We also describe a diagnostic method carried out by a system of any embodiment, the method comprising the steps of:
introducing a sample into the lysate stage and lysing the sample in the lysate stage to provide an analyte, or introducing a previously-lysed analyte into the device;
conveying the analyte through the assay stages within the device wherein movement and temperature of stages of the reader unit are controlled, and
detecting analyte at the sensor.

Preferably, heat is applied to the lysate stage in order to melt a wax plug, resulting in an outlet port of the lysate stage becoming unblocked.

Preferably, the beads are released from the wax plug, and enter the stage main body. Preferably, magnetic beads bind and capture target NA of the blood sample, in the lysate stage.

The lysate stage may contain transport magnetic beads (T-beads), PNA probes, and target nucleic acid. Preferably, the magnetic drive moves so as to magnetically move the beads from the lysate stage to a wash assay stage.

Preferably, in the wash assay stage, cellular debris and contaminants are removed from the sample. Preferably, if the target nucleic is present, a T-bead (transport bead) R-bead (reporter bead) tethered sandwich is formed in an assay stage.

Preferably, the magnetic drive moves so as to magnetically move the beads from the wash assay stage to a reporter bead assay stage.

Preferably, the reporter bead assay stage contains smaller 'reporter' beads (e.g. 0.5µm), which have attached a second PNA-probe ("Probe2") complementary to the target nucleic acid, and if the target NA is present, a T-bead R-bead tethered sandwich is formed within the reservoir.

Preferably, the magnetic drive moves so as to magnetically move the beads from the reporter bead assay stage to a reporter bead wash assay stage. Preferably, in the reporter bead wash assay stage, any excess R-beads are removed resulting in a 1:1 ratio of R-beads and NA targets.

The magnetic drive may move so as to magnetically move the beads from the reporter bead wash assay stage to a sensor assay stage.

The sensor may comprise a capacitive sensor.

Preferably, in the sensor assay stage, heat is applied to the reservoir to separate the T-beads. Preferably, T-beads are magnetically moved to a waste reservoir before sensing.

Excess liquid may be evaporated and transported to a separate reservoir.

The sensor may detect capacitance. Preferably, a capacitance reading corresponds to reporter beads on the sensor surface. The reading may for example indicate the presence of a viral infection.

The sensor may be functionalized with for example PNA probes.

Preferably, neutral beads are provided which play no part in molecular binding but provide for stabilisation to help the transport beads break the interfacial tension by their greater mass and responsiveness to the external magnetic field.

The lysis stage may contain transport beads, and said beads travel out of the lysis stage and into a channel microfluidic containing molten wax in a contiguous column which has connection to aqueous reservoirs and the interfacial tension and capillary force keep assay stage reservoirs isolated from each other.

Preferably, PNA and transport beads are microencapsulated (e.g. ethyl cellulose, polyvinyl alcohol, gelatin, sodium alginate) prior to being incorporated in the wax of the lysis stage.

The lysis buffer may be adjusted so as to only cause lysis in certain target cells or capsids in the sample, and optionally this adjustment involves a drug to bind to a particular protein target on the viral surface which damages membrane integrity.

Preferably, the probe also contains cell-penetrating peptides (e.g. trans-activating transcriptional activator TAT) to facilitate cellular uptake prior to lysis, limiting exposure of the NA target to nucleases prior to lysis.

A heater may be adjusted to specific temperatures so as to only cause lysis of certain types of cells or capsids in the sample.

Preferably, the adjustment includes applying a stress such as heat to all cells within a blood sample wherein white blood cells (containing DNA) can respond to prevent lysis whereas other cells and, particularly, viral particles are lysed.

Preferably, a blood sample passes through immunomagnetic beads suitable for removing certain fractions of the blood prior to application of the sample to the device (e.g. Whole Blood MicroBeads Miltenyi Biotec).

Preferably, blood passes through a bed of resinous beads which may be functionalised (e.g. Chelex) or coated in reagents to help prevent clotting (other chelating agents such as EDTA or citrate).

Preferably, these beads are functionalised with ligands for selective capture of certain fractions of the blood after application of the sample to the device.

Preferably, the beads have a density such that density and gravity will hold these *in situ,* particularly where this section is vertical.

Organic solvents may be used such that low density allows gravity to assist R-beads to drop across or down on to the sensor faster due to their lower buoyancy.

Preferably, multiple NA targets are targeted so that two or more types of R beads arrive on the sensor, and the sensor determines the quantities of each type of R bead.

PNA and transport beads may be microencapsulated (e.g. ethyl cellulose, polyvinyl alcohol, gelatin, sodium alginate) prior to being incorporated in a wax.

Preferably, reporter beads are produced using a fluidic system to be doped with materials such as Titania or Barium Titanate to give a strong signal to a capacitance sensor.

PNA probes on the sensor may be complementary to a standard PNA probe on each type of R bead, and the R beads are additionally functionalised with HIV_Probe2 and HCV_Probe2. As the sensors come manufactured with standard PNAs, this approach allows more rapid assay development.

Preferably, transport beads which are magnetic and buoyant in water are manipulated using external magnetic fields within solutions less dense than water (e.g. 20% ethanol).

Preferably, the method includes multiplexing of analytes in the device.

Multiple targets may be targeted in parallel by providing a plurality of types of reporter beads in an assay stage so that a plurality of two types of reporter beads arriving on the sensor and the sensor determines the quantities of each type of reporter bead.

Preferably, the sensor comprises at least two sensor regions with PNA probes which are complementary to PNAs on the R beads so that each type of R bead becomes hybridised to the correct sensor.

The sequence of steps for an assay may be driven by a processor of the device.

Preferably, the device incudes a stored and preferably encoded identifier for a sample which it has received and this is communicated either by physical terminals or wirelessly by the device to the reader.

Preferably, the identifier is correlated with a device or sample identifier physically printed on the device housing, such as in a code such as a 1D code or a 2D code.

### Detailed Description of the Invention

The invention will be more clearly understood from the following description of some embodiments thereof, given by way of example only with reference to the accompanying drawings in which:-
Fig. 1 is a diagrammatic plan view of a diagnostic device;
Figs. 2 and 3 are diagrams illustrating bead binding;
Fig. 4 shows a lysate vessel of a device of another embodiment;
Fig. 5 is a perspective cut-away view of a device in the form of a cartridge for mounting vertically in a reader;
Fig. 6 is a perspective cut-away view, and Figs. 7 and 8 are front and rear perspective views of an alternative cartridge;
Figs. 9A and 9B show the effect of a hydrophobic coating on a channel for shape of an oil-water interface;
Fig. 10 illustrates the cartridge being inserted into an automated portable diagnostic reader of a system of the invention;
Figs. 11 to 14 show the cartridge in the reader during use; and
Fig. 15 shows an alternative device.

### Description of the Embodiments

### Overview

A diagnostic device is in the form of a cartridge for inserting in a reader which provides for movement and heating of analyte, the reader and the cartridge providing together a diagnostic system. The system is such that the disposable cartridge which has a processor, controls the reader unit, simplifying the reader unit and maintenance thereof.

It is also envisaged that a device may be used to complete an assay without an automated actuation system, with analyte movement and/or heating being performed manually by a skilled operator. However a reader linked with the sensor would be required wither as part of the device or separately.

The device is in one example for taking a sample of blood, lysing the sample to release an analyte such as DNA, RNA, proteins, or extracellular vesicles, and tagging the analyte to magnetic transport beads (T-beads). In other examples, the sample is processed or lysed beforehand.

The whole blood sample may have a suitable diluent added, such as DI. Hereafter, blood sample is taken to mean either a whole blood sample or diluted whole blood sample.

In one example, a covalently-attached PNA (peptide nucleic acid) Probe1 on a T-bead captures an RNA (Ribonucleic nucleic acid) target. This is achieved by transport beads ("T-beads") magnetically moving the analyte through a series of assay stages within the device. An external magnetic field is applied to the device so that the T-beads move magnetically and sufficiently treat the analyte so that a diagnostic reading can be taken of the analyte.

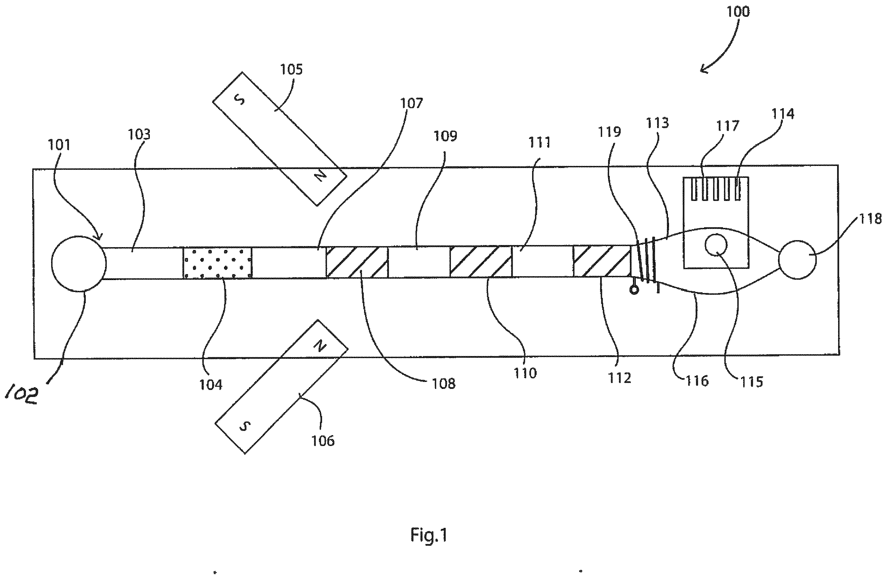

Referring to Fig. 1 a device 100 of one embodiment is illustrated. The following are the components
- 101: plastic assay tube having an internal diameter of 0.6mm,
- 102: blood inlet,
- 103: lysis chamber,
- 104: solid wax and bead matrix plug,
- 105, 106: dipolar magnets,
- 107: first wash chamber,
- 108: first oil barrier, (166)
- 109: PNA Probe2 attachment chamber,
- 110: second oil barrier,
- 111: second wash chamber,
- 112: third oil barrier,
- 113: sensing chamber,
- 114: CMOS chip,
- 115: capacitance sensor on the chip 114,
- 116: microfluidic capsule,
- 117: contact pads or fingers, in this case gold-plated,
- 118: removable rubber plug, and
- 119: induction coil heater

In more detail, the device comprises a simple microfluidic tube (101) with a series of assay stages including the lysis buffer 103, the aqueous wash buffers 107 ad 111, and oil/wax barriers 108 and 110 through which the magnetic beads travel.

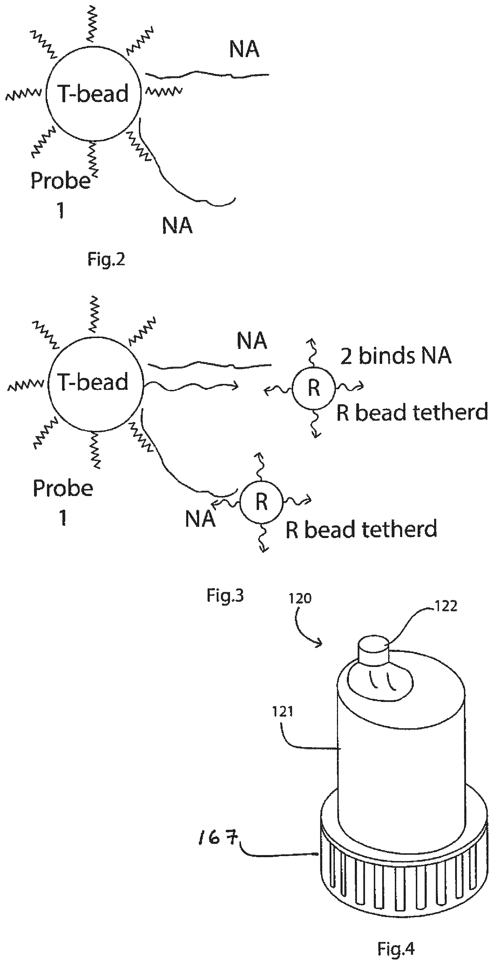

A hot plate underneath the device is used to assist lysis of the whole blood and/or any viral envelopes or capsids present in the sample. This act of heating the lysis section also melts the wax and bead matrix plug 104. This wax/bead matrix contains magnetic beads ("transport beads" or "T-beads") of ∼1µm in diameter. The beads have a coating such as a covalently-attached PNA probe(s), selected for capturing a specific target NA analyte. Please refer to Fig. 2, in which the attachment of Probe1 to a T-bead is shown, with two strands of captured NA shown.

The device is in one example for taking a sample of blood, lysing the sample to release an analyte such as DNA, RNA, proteins, or extracellular vesicles, and tagging the analyte to magnetic transport beads (T-beads). A covalently-attached PNA (peptide nucleic acid) Probe1 on a T-bead captures an RNA target. This is achieved by transport beads ("T-beads") magnetically moving the analyte through the series of assay stages within the device 100. An external magnetic field is applied to the device by the magnets 105 and 106 so that the T-beads move magnetically and sufficiently treat the analyte so that a diagnostic reading can be taken of the analyte.

It will be appreciated that the application of an external magnetic field collects the T-beads, and causes them to break through the interfacial tension between aqueous lysate and a now molten wax layer. It is preferred that at least one of the magnets is a conical focal magnet, for optimum control.

A small volume, for example 10µl, of whole blood is applied to the inlet 102 (directly or using a pipette). A small plug is placed over the inlet 102 to seal the device. A lysis buffer in the tube segment 103 mixes with the blood, causing lysis to occur.

In another embodiment, the PNA and T-beads are microencapsulated (e.g. ethyl cellulose, polyvinyl alcohol, gelatin, sodium alginate) prior to being incorporated in the wax. This may provide for better stability and controlled release of the particles during the assay.

Once the wax plug is melted the beads are pulled magnetically by the two opposing external magnets 105 and 106 (either manually or robotically-operated) towards the left as shown in Fig. 1 into the now-lysed blood in the lysis reservoir 103. The covalently-attached PNA probes on the surface of the magnetic T-beads hybridises to and captures target NA within the lysate, as shown in Fig. 2. Small circular movements of the external magnets on either side of the tube promotes active mixing and increased binding and capture efficiency by the beads. Thus, a target NA becomes attached to Probe 1 which in turn is covalently attached to the T-bead.

In one embodiment the lysis buffer can be adjusted so as to only cause lysis in certain target cells or capsids in the sample. This adjustment may for example involve a drug designed to bind to a particular protein target on the viral surface which damages membrane integrity.

In one embodiment, the probe also contains cell-penetrating peptides (e.g. trans-activating transcriptional activator TAT) to facilitate cellular uptake prior to lysis. This limits exposure of the NA target to nucleases prior to lysis. This would be particularly beneficial in the case of PNA probes wherein nucleases do not recognise the PNA-NA duplex. The probes could be bound to particles or biotinylated for capture by streptavidin-coated beads.

In one embodiment the hot plate can be adjusted to specific temperatures so as to only cause lysis of certain types of cells or capsids in the sample. In one example, the stress induced by such a temperature adjustment may cause white blood cells (containing DNA) to respond, preventing their lysis, whereas other cells and, particularly, viral particles which cannot respond to these temperature variations are lysed. This is advantageous where the white blood cells contain retroviral DNA which can confound an assay for retroviral RNA. This retroviral DNA will not be available within the lysate produced here.

The T-beads with attached NA target are now magnetically aggregated using the external magnets, and pulled back though the melted wax plug (104). This wax acts as a barrier which separates the biological sample from the rest of the assay. As the beads pass into the wax they interfacial tension and entropy causes them to form into a tight, aqueous ball of beads (∼0.5mm diameter), thereby, limiting the amount of non-targeted biological contaminants. This ball of beads moves easily in the oil/wax and control of its movement (including around obstacles) is facile.

### First Wash Stage

Using the external magnets 105 and 106, the T-beads, with concentrated and enriched NA attached are pulled into the aqueous wash reservoir 107. Here, the T-beads are actively mixed for 10s using the external magnets to release any non-target biological material which may have been carried thought the wax barrier. The T-beads are then once again aggregated and pulled through the first oil phase 108.

### Reporter Probe Attachment Stage

Once washing of the analyte has been completed, the magnets 105 and 106 move the washed T-beads to the next assay stage, through the first oil barrier 108 into the reservoir 109. This oil can be a low-viscosity oil such as silicone through which the beads can particularly easily travel. The reservoir 109 contains a PNA probe (Probe2) which is complementary to a different section of the target NA than the original Probe1. In this embodiment the reporter Probe2 is covalently attached to a reporter bead (R-bead).

These R-beads are significantly less magnetically susceptible than the T-beads, either by being much smaller in diameter than the T-beads (0.1 µm to 0.5µm vs 1.0 µm) or containing fewer superparamagnetic nanoparticles for a given mass or May be made of a non-magnetic material.

The R-beads can be produced using a fluidic system to be doped with materials such as Titania or Barium Titanate to give a strong signal to a capacitance sensor.

Using the external magnets, T-beads are mixed within this reservoir. If any target NA is present on the T-beads they bind and become attached to PNA Probe2 on the R-beads. This binding of the two probes across the same NA results in the T-beads and R-beads being tethered together, as shown in Fig. 3. A T-bead-R-bead tethered sandwich is now formed, only if the NA target is present. The sandwich comprises: T-bead - Probe1 - target NA - Probe2 - R-bead.

T-beads are once again magnetically aggregated and removed from the reservoir 109 though the second oil barrier 110 and transported into the aqueous reservoir (e.g. DI) 111 which forms the second wash stage.

In another embodiment Probe2 is biotinylated so that a positive capture result can be observed through capture of Streptavidin conjugated horseradish peroxidase. Using a similar liquid-liquid purification process, when these are selectively transported and provided with the substrate Tetramethylbenzidine (TMB), a colorimetric change is observed indicating the presence of target NA (e.g. HIV).

In one embodiment Probe2 has a fluorescent moiety or particle attached which can be detected using a plate reader etc.

### Second Wash Step

Once the T-beads have been moved into the reservoir 111 by the external magnets, the beads are once again mixed and separated into solution, this time to release any non-attached R-bead which may have been carried through from the previous capture phase. The T-beads are then aggregated once again and pulled through the oil barrier 112 into another reservoir 113 which includes a semi-conductor sensor 115 on a chip 114. There is now a 1:1 ratio of R-beads to NA targets. This is because the R-beads can only get from the reservoir 111 to the reservoir 113 if tethered to the T-bead by the Probe1 - target NA - Probe2 sandwich.

The T-beads are now magnetically moved over the surface of the sensor 115. Heat is applied in order to melt the PNA-Probe2-NA links and separate the T-beads from the analyte NA and hence separate the R-beads. The magnetic T-bead can now be pulled away, leaving the much less magnetic (or non-magnetic) R-beads over the sensor surface. The T-beads are moved back through the oil barrier 112 as they are now no longer required, and should they remain in the reservoir 113 may affect the capacitance reading on the semi-conductor chip.

The Probe2 heating temperature to separate the second probes is typically 60°C to 85°C, depending on its exact hybridization sequence. This temperature may be achieved by placing the device substrate on a hot plate, and/or or by using an induction coil 119, which enables magnetic induction heating of ferrite R-beads, as described in US5,378,879.

In one embodiment the reservoir 113 is filled with ethanol. Attached to the reservoir is a Fluorosilicone (or other ethanol resistant material) plug 118 which when removed allows the ethanol within to evaporate.

### Multiplexing

It will be understood that multiple NA targets could be targeted in parallel using the above approach, e.g. for detecting two types of virus at the same time (HIV_T-bead - HIV_Probe1 - HIV_target NA - HIV_Probe2 - HIV_R-bead; HCV_T-bead - HCV_Probe1 - HCV_target NA - HCV_Probe2 - HCV_R-bead). This results in two types of R beads arriving on the sensor. The sensor can determine the quantities of each type of R bead.

The sensor may have at least two sensor regions with PNA probes which are complementary to PNAs on the R beads so that each type of R bead becomes hybridised to the correct sensor.

The PNA probes on the sensor may be complementary to the HIV_Probe2 and HCV_Probe2.

Alternatively, the PNA probes on the sensor may be complementary to a standard PNA probe on each type of R bead. These R beads would then be additionally functionalised with HIV_Probe2 or HCV_Probe2. As the sensors come manufactured with standard PNAs, this approach allows more rapid assay development.

Ideally, the standard PNAs should be designed to have no interaction with NAs found in nature and have good robustness to chemical and heat treatments found during wafer-level manufacture (e.g. through careful design of the sequences).

### Sensing Stage

The sensor chip 114 is a CMOS semiconductor capacitance sensor chip and is mounted in the encapsulated microfluidic structure at the end of the assay tube 101. It contains a high-resolution sigma-delta capacitance-to-digital converter, calibration memory, and digital processing circuitry, including I2C serial communication. A protrusion 117 with four gold fingers (VDD, GND, Sclock, SData) connect the chip to an external reader or computer. A first "wet-capacitance" reading is taken by the sensor chip, of the R-beads in liquid. This provides a baseline reference calibration capacitance. After the liquid evaporates, a second "dry capacitance" reading is taken of the R-bead capacitance. Due to their dielectric constant being higher than air, any R-beads on the sensor surface give a capacitive signal. e.g. IfF (delta capacitance versus dry air) for 200 beads, as shown in Fig. 19 of WO2015086654.

In simple embodiments, interdigitated electrodes on the sensor can be arranged to act as a "snag trap" for R-beads. This may be accomplished by orienting them perpendicular to the flow of beads and controlling the pitch of the electrodes so that only the small R beads can fit between them.

Also, it will be appreciated that the sensor capacitive signal represents the sample target NA, due to the 1:1 ratio of R-beads to target NA. This indicates a positive qualitative detection of the target NA. With suitable controls, this is clinically very significant as it provides a true or false indication of whether the sample being tested contains the target RNA virus.

The value of this capacitive reading corresponds to the number of R-beads on the sensor surface -which corresponds proportionately to the number of target RNAs in the original blood sample. This is indirect quantification of the number of copies of RNA per unit volume of the original sample, i.e. the assay is both qualitative and quantitative. This number of RNA copies (the "viral load") is also clinically very significant. It enables a physician to better diagnose a patient's precise health condition, and prescribe the correct treatment drug to a patient.

In another embodiment, addition of a drop of toluene degrades the polystyrene coating of the beads, deforming them, so the ferrite core lodges nearer to insulated electrodes, giving a further increase in capacitive signal and assay sensitivity.

### Lysis Vessel

In another embodiment, a lysate vessel (100u1-1ml) can be affixed to the end of the assay tube to provide a lysate stage instead of the tube segment 103. This lyses the sample, for example as described in WO2015086652, for example by heating at a temperature in the range of 60°C to 99.5°C for a time duration in the range of approximately 2 to 5 minutes.

Referring to Fig. 4 such a lysate vessel 120 may have a main body 121 and a funnel-shaped outlet 122 whish retains a solid plug of wax, and this must be melted by the heater to allow passage of lysate into the first stage within the conduit. First probe attachment may occur in this chamber.

It will be appreciated that the application of an external magnetic field collects the T-beads, and a conical focal magnet may be used to allow the plug of beads to break through the interfacial tension between aqueous lysate and a now molten wax layer. The magnets may be bar or disc shaped, or also may be conical in shape, and it has been found that this shape is very suitable for accurate manipulation of the magnetic beads in channels, as the field lines are concentrated at the magnet apex. This can concentrate the beads within the channel into a small area, move them effectively against interfacial tensions between aqueous solutions and wax separation plugs or oil within the cartridge, so that they 'walk' through the assay steps of enrichment, purification, washing, tethering, and sensing, under magnetic control.

The use of two opposing magnets, 104 and 105, greatly assists bead movement, mixing, and dispersal, by creating a steep gradient in the magnetic field and particle repulsion between the two opposing North poles. This mixing and homogenising of the beads within an aqueous solution provides excellent assay kinetics, and good overall assay time. The steepest magnetic gradient point is achieved by moving the two magnets away from the channel, to a distance of approximately 6mm to 12mm. Both magnets may be moved simultaneously or one magnet may be moved at one time. Rotating or oscillating one magnet back and forth against the other static magnet also effects good mixing.

This device is very suitable for rapid assay development, for simple assays, using flat-plate heaters, and magnets for bead movement and control.

### Viral Load Quantification

The following describes a range of approaches from a simple way to deal with the specific problem of retroviral DNA through to a more complicated way. They all provide a liquid sample which can be whole blood through to serum.

Some viral targets (e.g. retroviruses) incorporate themselves as DNA in human cells. To distinguish such nucleic acid targets from cell free RNA within virions, the assay may also rely on the higher melting temperature of RNA-PNA, compared to DNA-PNA. This can be several degrees higher depending on sequence and enables differential melting with suitable control of temperature, to assist separation of cell-free RNA.

Some embodiments may include fractionation of a whole blood sample. The simplest embodiment involves centrifugation of a tube of whole blood and application of the virus containing serum component to the inlet. In another embodiment, a filter which is selective for white blood cells (containing the retroviral DNA) is applied (Acrodisc® WBC Syringe Filter) is used and requires only gravity in a 10-15min incubation step. The filtrate containing only the viral particles, red blood cells, platelets produced is inputted to the device. In another embodiment, a simpler filter (Novolytic, Indira) is applied which removes all blood cells. This may require higher back pressures.

Alternative embodiments incorporate syringes with attached filter systems (Novolytic, Indira). The syringe may filter all blood cells to provide a serum sample for the device. US 5,139,685 and US 6,391,265 are two prior art examples of using a membrane filter in a device.

In other embodiments, the blood passes through immunomagnetic beads suitable for removing certain fractions of the blood prior to application of the sample to the device (e.g. Whole Blood MicroBeads Miltenyi Biotec).

The liquid sample can be added to the device with any suitable needle or pipette tip.

In another embodiment, the sample can be applied by piercing a membrane seal 167 with any suitable sample-containing receptacle (e.g. syringe, needle, capillary, plastic dropper, pipette tip).

In another embodiment, the blood passes through a bed of resinous beads which may be functionalised (e.g. Chelex) or coated in reagents to help prevent clotting (other chelating agents such as EDTA or citrate). In some embodiments, these beads could also be functionalised with ligands for selective capture of certain fractions of the blood after application of the sample to the device (following same principle of Whole Blood MicroBeads Miltenyi Biotec). In some embodiments, these beads are high density and gravity will hold these *in situ,* particularly where this section is vertical.

In other embodiments, the blood is lysed and the lysate passes through a static matrix of T-beads wherein the analyte is captured.

In other embodiments, the lysate may be passed through a simple filter (<1.0um pore size) which filters out aggregated material (lysed cellular membranes/protein) while allowing a filtrate containing an analyte (e.g. RNA) to pass. The analyte is captured on 1.0um diameter T-beads, which cannot pass through the filter in the opposite direction.

In another embodiment, the T-beads are held away from the aggregated material (e.g. by magnetic fields).

### Automated Cartridge and Reader system (160)

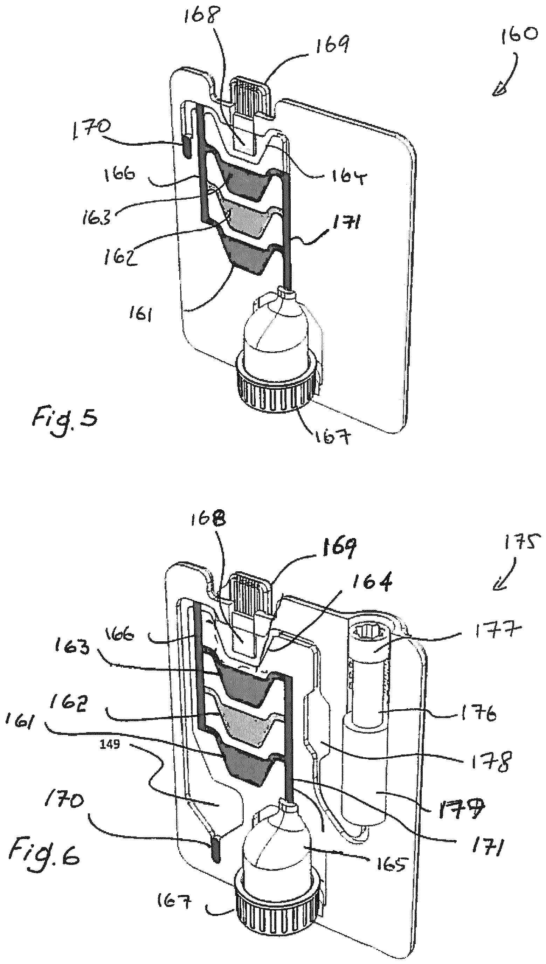

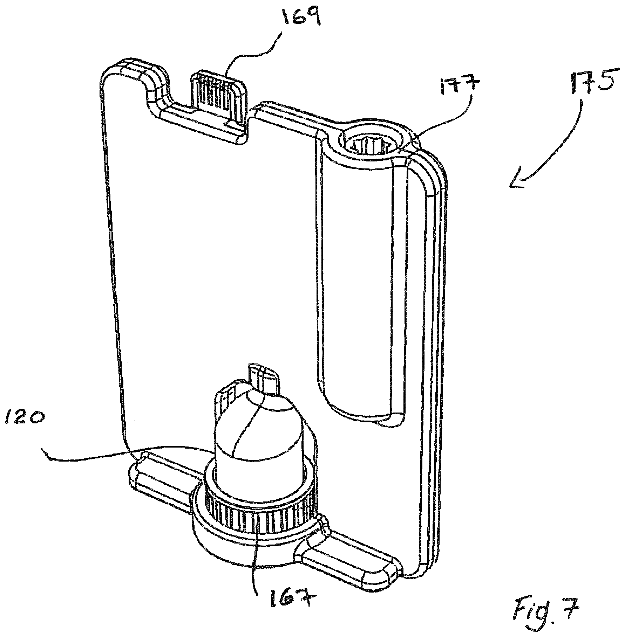

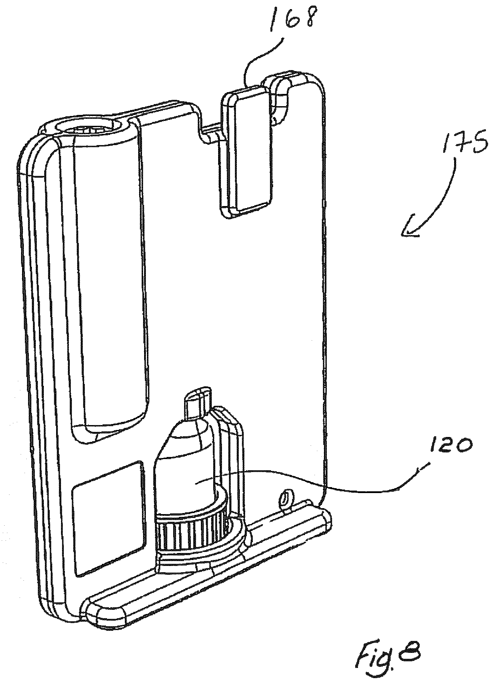

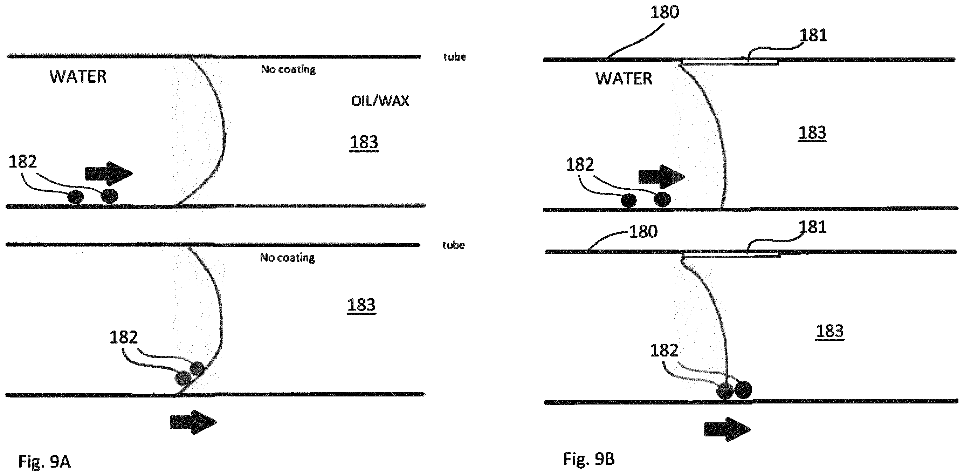

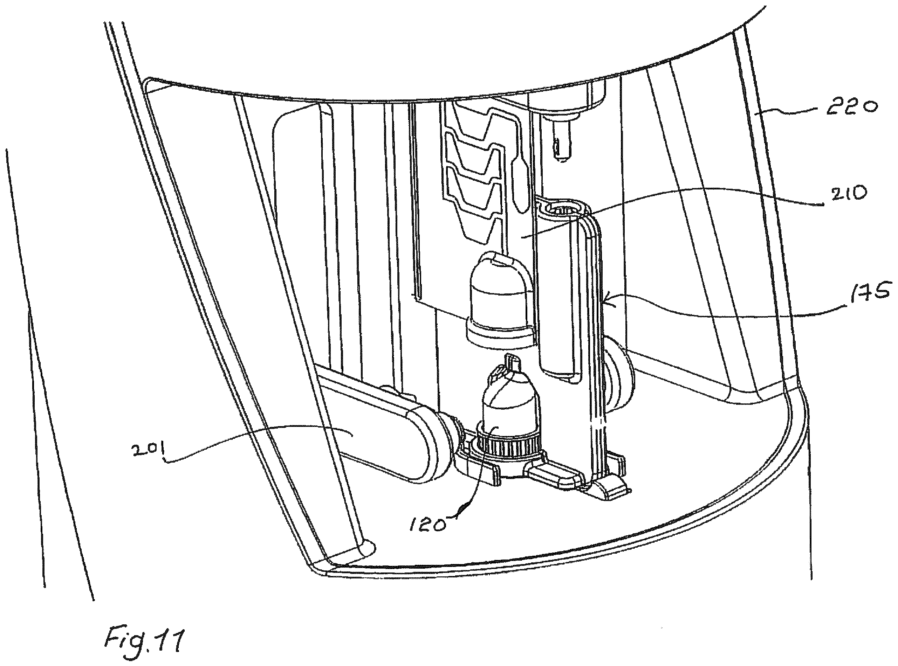

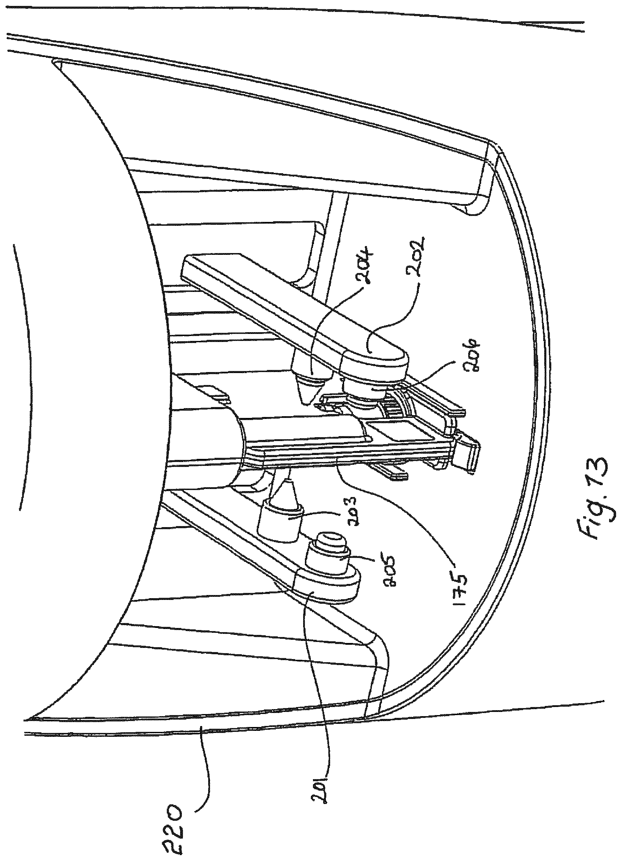

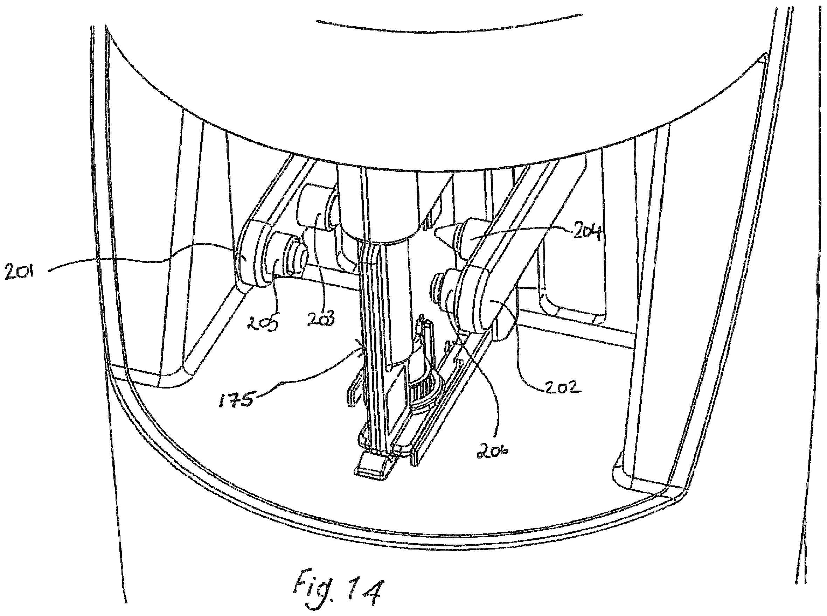

Fig. 5 shows a device 160 and Figs. 6 to 8 show a device 175, which are two embodiments of injection-moulded fully integrated, sealed cartridges for insertion into a reader unit 200, as shown in Figs. 9 to 14. Like parts of the two devices are indicated by the same reference numerals. Reservoirs 161, 162, 163, 164 are formed and joined by channels 166 and 171 through which the beads travel. The channels are vertical; they may be coated with Teflon™. Corners are rounded, to facilitate smooth bead movement. The microfluidic interfaces are designed with smooth, narrowed, vertical inlets and outlets to maintain the integrity of oil/water barriers across these even where the device is tilted.

In summary the major parts are:
- 161: first wash chamber,
- 162: PNA Probe2 attachment chamber,
- 163: second wash chamber,
- 164: sensor stage
- 165: solid wax and bead matrix plug,
- 166: second oil barrier,
- 167: blood inlet,
- 168: CMOS chip, and
- 169: contact pads or fingers, in this case gold-plated.
- 171: first and third oil barrier.

The device 175 has the following additional components:
- 149: waste region,
- 170: air filter,
- 176: piston,
- 177: piston actuator,
- 178: wash buffer reservoir, and
- 179: gas expansion chamber.

As may be noted from the above, the device allows oil barriers 165 and 171 to be used multiple times. This advantageously allows for a more compact design, simpler assembly and operation. To highlight this, the device 175 has two contiguous oil/wax areas (165/171 and 166) as opposed to the four such areas in the linear device 100 (104, 108, 110, 112).

There is a need for precise positioning of the cartridge to provide for mechanical movements and/or automation of the steps. This is referred to above in the design of the cartridge to be rigid with the design allowing for a system of locks to ensure precise positioning of the system relative to the reader.

In some embodiments, hydrophobic coatings (e.g. Aculon) may be applied to surfaces of the microfluidic 175 at the position of the interface of oil/wax and water to strengthen the barrier to aqueous solution.

As beads are pulled towards the oil/water meniscus (Fig. 9A) by a magnetic field applied to the bottom of the tube (as shown), it's noted in the bottom sketch of Fig. 9A that the beads will be deflected up by the shape of the meniscus at this point as the hydrophilic beads prefer to stay in the aqueous solution. As the beads move upwards away from the magnetic field, the strength of said field is reduced. This can make it more difficult for the beads to break the meniscus. In WO2015086652A1, the magnetic field is applied through the centre of the oil/water meniscus meaning the gradient increases towards the centre of the meniscus, where it is easiest for the bolus of beads to collect and break through the meniscus. As shown in Fig. 9B, in other embodiments, a hydrophobic coating 181 is applied to one wall 180 of the microfluidic at the location of the wax (or oil) 183. This changes the shape of the oil/water interface, as illustrated in Fig. 9B as water is repelled at that surface. As magnetic beads 182 approach, they encounter a smaller contact angle due to the hydrophobic coating 181. This changes the angle of incidence of the meniscus at the oil/water barrier, making it easier for the bolus of magnetic beads 182 to pass along the untreated microfluidic wall. The diagram of Fig. 9A shows the situation without a hydrophobic coating, showing diagrammatically a large angle at the surface and beads finding it more difficult to pass through the meniscus as they are deflected upwards.

In preferred embodiments featuring the capacitance chip in the sensor, the chip needs to be carefully and mechanically inserted into the reader unit via a micro USB or other connection. In preferred embodiments, the cartridge is locked into the cartridge and the chip inserted vertically to provide for ease of access of the two robotic arms and heaters and the mechanical screw drive. Simple commercial point of care systems rely on passive flow systems such as capillary action and/or lateral flow strips. These have inherent weaknesses for generation of complex data in failing to provide for precise control of the assay and fluidics. More complex systems rely on elastomeric elements, multiple valves and complex external manipulation using up to nine actuators, and a separate rigid "exoskeleton" required for precise positioning (e.g. US7,718,421).

The device minimises the risk of user error, is largely mechanical, and inexpensive. It requires little training to use.

The device is very well aligned with what is needed to achieve a CLIA (Clinical Laboratory Improvement Amendments) waiver and, with the sensor providing excellent control of data input and output, could even be an improvement on laboratory equipment where there are still manual elements such as barcode scanning/data recording.

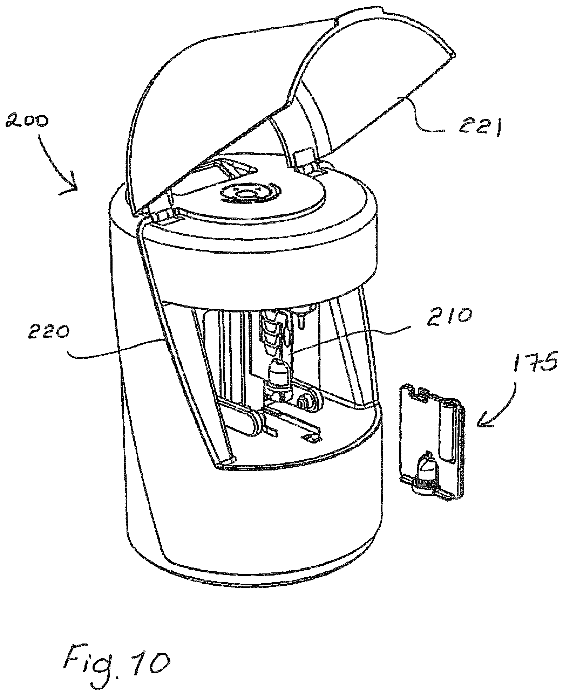

Referring also to Figs. 10 to 15, a reader 200 has an opening 220 which may be closed by a cover 221. Opening the cover 221 reveals a support mechanism for the cartridge 175, comprising a support element 210 which generally conforms to the shape of part of the cartridge 175. In the reader 200 magnetic force is applied to the cartridge 175 by reader unit magnets 203, 204, 205 and 206 on movable arms 201 and 202, respectively. The magnets Neodymium X12X12CON™ may be conical-shaped magnets (203 204) for focal movement of beads or disc-shaped (205 206), for bead mixing, according to the assay stages described above.

Heat is applied to the cartridge 175 by the thin heater element overlay 210 which covers the cartridge 175 in the space between the movable arms 201 and 202 and the exterior surface of the cartridge 175. An example is the Watlow Flexible silicone rubber heaters which have rapid heat transfer, operating temperatures to 260C, Watt densities 12.5 W/cm2, 0.5mm thick with an etched foil element. These are suitable for medical equipment such as blood analysers and test tube heaters. The overlay 210 heater elements are incorporated so as to be in registry with the stages of the device 175.

The reader 200 is controlled by a microprocessor linked with terminals which engage with the terminals of the sensor 168 of the device 175 when inserted.

In other embodiments, point-heating may also be applied by heater elements incorporated into the movable arms 201 and 202.

There also may be a heater element underneath a lysate vessel 120, adjacent the flat portion of the vessel.

The chip 168 is mounted on a protruding connector 169 with four gold fingers (VDD, GND, Sclock, SData), for electrical mating with a connector in the reader unit. A heater and temperature sensor on the chip 168 may also facilitate heating and melting of Probe-2 in the reservoir 164, with real-time feedback and assay monitoring by the temperature sensor, for precise assay temperature control around the melting point. This is a major advantage of using a disposable chip within the cartridge. The chip 168 is immersed and sealed in the microfluidic cartridge. The chip has multiple sensors - 32 sensors in this embodiment.

The 32 sensors have 32 PNA probes immobilised on their surface by spotting during chip or cartridge manufacture. R-bead hybridisation to the surface occurs as per the assay described for the multiplex assay earlier, however there are up to 32 targets rather than two. There are therefore 32 sets of PNA probes in the assay.

The assay proceeds as outlined in the Multiplexing assay above.

After R-bead separation, the T-beads are removed to a waste area 149.

In some assay embodiments the R-beads may be non-magnetic (e.g. gold or oxide nanoparticles), which also facilitates this separation.

A column of liquid over the sensor chip in the reservoir 164 is gently moved over and back across the sensor surface by gentle oscillation and pressure of a piston 176, driven by a screw-actuator 177 which engages with the external reader unit. This causes gentle liquid mixing of the beads during the incubation period (5-30 mins), and facilitates bead tethering to 32 sensors. The number of R beads attached to each sensor is proportional to the number of RNAs in the sample initially. After tethering, liquid is then removed from the sensor surface, by pressure of the piston 176, into an expansion chamber 179 which contains air (or dry nitrogen). A wash solution 178 is pushed ahead of the gas over the sensors. When the gas is over the sensors, a 'dry' capacitance reading is taken as before, and a lower-limit-of-quantification of less than 100 beads

A waste region 149 occurs into which the displaced liquid (and molten wax; chip reservoir buffer; surfactant; and any gas) may be pushed. This is precisely designed to contain a volume of sterile gas which is pushed in front of this liquid through a HEPA filtered vent 170 to the environment. This equilibrates the pressure within the cartridge and allows easier tolerances within the design and manufacture of the cartridge.

The only connection of the cartridge to the environment is filtered and only air is expelled through it. All biological material (even including the target RNA) is retained within, which makes this cartridge safely disposable. The lack of any fluidic connection to the reader is a major advantage of the invention.

In another embodiment, a collapsed elastomeric/membrane region within the vent provides for pressure equilibration which does not involve any connection to the environment and thereby the cartridge is completely sealed during and after use.

The device 160 of Fig. 5 has a final reservoir 164 containing an organic liquid with a much lower dielectric constant than water (e.g. ethanol). Where there are large numbers of R-beads and the R-beads are magnetic, these may be moved on to the sensor and the R-beads measured against the organic liquid rather than air (dielectric constant of 1). No drying is performed.

Similarly, in a multiplex embodiment, the cartridge 160 may have a final reservoir containing an ethanol solution. PNA-PNA binding is possible in this scenario and, as such, the multiplexing assay can be accomplished. An organic solvent can be removed by allowing evaporation or active heating.

In embodiments containing organic solvents, it will be clear that the lower density allows gravity to assist R-beads to drop across (vertical configuration) or down (horizontal configuration) on to the chip faster due to their lower buoyancy in the alcohol solution. This is desirable for some applications.

In another embodiment, T-beads, which are magnetic and designed to be buoyant in water, can be manipulated using external magnetic fields within solutions less dense than water (e.g. 20% ethanol). Consequently, T-beads can still be removed from the reservoir above the chip. However, an R-bead which is buoyant in aqueous solution and which is untethered from a T-bead above the chip will have an increased tendency to sink on to the surface of the chip. This is desirable to promote rapid sinking of the R-beads on to the surface of the chip in this embodiment. A reader which supports the cartridge horizontally may be used.

### Actuation System

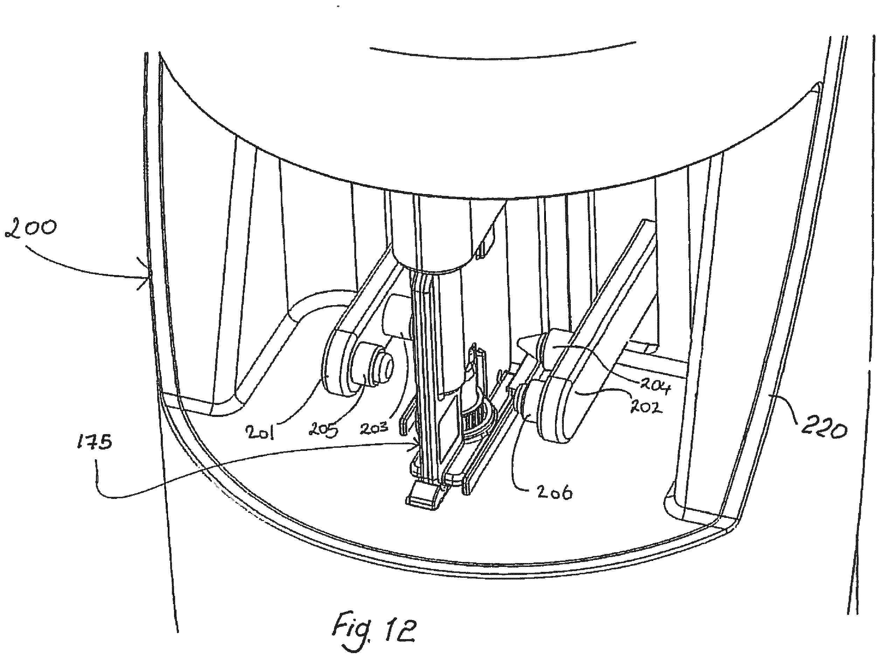

Referring again to Figs. 12 to 14, the actuation system mediates engagement of external magnetic fields is a sub-assembly within the reader. One embodiment involves the use of linear actuators for precise movement of an arm mechanism in the X-, Y- and Z-axes. The arm is branched in two, one on either side of the cartridge system, and set back about 2cm from the LEFT and RIGHT sides of the cartridge system, respectively. On the arms there are mounted at least three permanent magnets, e.g. Neodymium Rare Earth Magnet Apex Magnets M12X12CON; M20Wdg; M121418diamR. These magnets are mounted in such a way on the arms so that the net magnetic field on the cartridge is minimal when they are set to their neutral position, equidistant from the centre of the cartridge. However, when the arm is actuated in the plus or minus direction in the X axis, the magnet on one or other arm is engaged and any magnetic beads within that field are drawn towards it. As the arm can also be moved in the Y- and Z-axes, beads can be moved accurately in three dimensions within the microfluidic.

In another embodiment, the arm of at least one actuator is connected to a commercially available actuation system as is used in an electric toothbrush, such as Philips Sonicare™. This sonication frequency (200-400 Hz) and complex sweeping movement provides for effective cleaning of dental surfaces in combination with a brush head. However, surprisingly, the underlying automated mechanism found within, when joined with a neodymium magnet, as above, provides for an unexpected mixing and homogenising effect on magnetic beads within a microfluidic. Improved kinetics for target capture are also possible. The costs of these actuators are low due to their mass manufacture for this common household item.

The movement of the arms 201 and 202 and thus the magnets 203 and 204, and heater elements on the magnets 205 and 206, is dynamically controlled by a programmed controller as required by the various stages of the system. This enables fully automated operation of the system by an untrained operator, once the blood sample has been taken.

Heaters incorporated into the magnets 205 and 206, and possibly also in the magnets 203 and 204 allow may operate as point heaters. These may heat through the magnets or around them, provided the heaters do not heat the magnets excessively (whereby they start to lose magnetism - the Curie temperature).

### Cartridge programs reader

In one example, the cartridge processor drives operation of the reader to perform the appropriate assay.

In a preferred embodiment, the cartridge processor and memory contains all of the information for a particular cycle of cooling, heating, detailed X-Y-Z actuator movement, incubation times or other data for controlling operation of the reader for performing the assay for which the cartridge is designed. This embodiment removes multiple steps seen in other point of care cartridge systems where the user must scan barcodes and select assays. In this embodiment the need for a graphical user interface is eliminated entirely, and replaced by a very simple LED "traffic light system" indicating to the user that a test has been successfully completed.

In one embodiment, a barcode can contain a reference ID for a particular cycle of cooling, heating, detailed X-Y-Z actuator movement, incubation times or other data that the system reader could reference. This will require database access for the reader to a corresponding set of information for each of these variables. This database will have to be comprehensive for each potential cartridge that could be used on the reader. In another embodiment, the cartridge chip contains the reference ID.

### Alternative Device Physical Conduit Arrangement

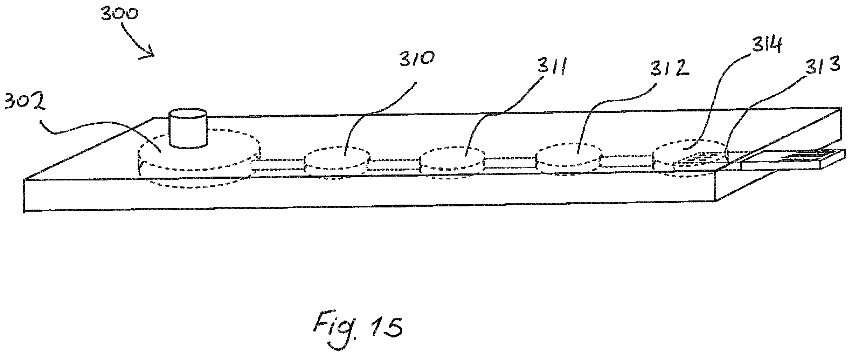

Fig. 15 shows an alternative assay cartridge physical arrangement of conduits and reservoirs, 300, suitable for 3D-printing construction. This has a lysis chamber 302 and stages 310, 311, 312, and 314 performing the same functions as the assay stages of the other embodiments, and terminating in a 3D-printed sensor electrode structure 313.

### Advantages of the invention

The invention does not require laboratories, refrigerators, or any complex laboratory equipment. The cartridge is completely sealed, with only an electrical connection to the reader. The sample and all reagents are retained internally, enabling very safe use and disposal. The reader unit can operate on a smartphone rechargeable battery for up to 12 hours. This enables RNA viral-load testing to be carried out without the need for a laboratory, and in small community and rural clinics, where it is often most needed, to rapidly intercept and diagnose disease outbreaks, and enable rapid point-of-care diagnosis and prescription of correct treatment, to stop further virus and disease spreading.

The assay employs synthetic PNA probes, beads, a semiconductor chip, oil/wax barriers, and DI water. The stability of these, and the lack of enzymes, proteins, and polymerases, makes the cartridge very robust and simple to manufacture. Logistical cost savings include removing the need for blue ice for transport and better shelf life (e.g. expensive stockpiles of PCR reagents for public health/biodefence tests are not required). The interfacial tension and capillary forces between the water and oil/wax barriers keeps the aqueous solution reservoirs isolated from each other. This replaces valving, greatly simplifying the design and manufacture of the cartridge

This also reduces complexity in the reader, saves on manufacture costs, and reduces the risk of device failure over time. It also enables wide temperature excursions of the cartridge during different parts of the assay (e.g. 90°C-95°C during a lysis step; 60°C during an RNA target annealing step; 90°C during an RNA melting step or PNA-PNA specificity step); - and wide temperature excursions during shipping and distribution, without any side effects. This is particularly important in eliminating dry-ice shipping requirements - a major barrier to widescale point-of-care diagnostic deployments.

Advantageously, the device also enables self-testing and calibration of the spotted sensors during wafer-level testing. The stability of the synthetic PNA probes is advantageous for wafer level processing which would be difficult for standard DNA or protein markers. This allows wafer level functionalization of the chip sensors prior to downstream testing, calibration, and processing. As the converters and digital calibration circuits in the CMOS sensor chip are located directly beneath the sensor pads, it allows for testing and calibration to be carried out easily, of the precise amount of probe spotting on each sensor, and recording of this for each sensor in an on-chip non-volatile memory. This is important for quantification of captured targets by the clinical end-user. This built-in self-testing and calibration capability is a major advantage over optical DNA microarrays, which can't be tested and calibrated, due to testing being destructive with optical probe attachment.

The cartridge could also work as a "smart" blood storage container. The patient ID is recorded on the chip EEPROM memory using a direct or wireless connection. The identifier can be further linked with a 2D barcode printed on the cartridge.

While we have described a cartridge for immediate analysis, it may be necessary for suitable additives to be included for blood storage within the lysis vessel 102 prior to further processing.

The ability to store the sample stably within the disposable system in a small format is advantageous for some end-user logistics.

In this embodiment, the user conducting collection needs to link the cartridge to the patient in a database.

This is done by scanning a 2D barcode on the cartridge; and by writing the patient ID to the EEPROM memory in the sensor chip within the cartridge, which can be transferred to another user/nurse for processing.

This may be logistically efficient in a primary health care setting where blood collection and processing could be both accomplished in the same facility by staff with little training.

The person conducting the processing does not know whose sample it is or even what test is being conducted, as this latter information is also stored on the chip and transferred to the reader automatically. This has important advantages for patient privacy.

In some embodiments, a lysate is applied directly to the device, without need for a lysate vessel.

It is envisaged that a reservoir may be filled with an organic solvent such as ethanol. Attached to the reservoir is a Fluorosilicone (or other ethanol resistant material) plug which when removed allows the ethanol within to evaporate. PNAs can bind in the presence of ethanol.

## Claims

1. A portable diagnostic device comprising:
a series of assay stages (161-164), each with a reservoir linked by channels (166, 171);
a lysate stage (165) for receiving a sample, said lysate stage containing magnetic beads with a probe and a lysis buffer, and comprising:
an outlet to the assay stages, and
a liquid-liquid purification means in the outlet and comprising a wax plug including said magnetic beads embedded in the wax plug for attachment of the probe to target analyte, providing for concentration and separation of an analyte from other material as it passes through the outlet; and
a sensor assay stage or an interface to a sensor for detecting beads attached to analyte molecules.

2. A portable diagnostic device as claimed in claim 1, wherein at least one assay stage (161, 163) is configured to carry out a wash, and there is a barrier (166, 171) in the channel between at least two assay stages, said barrier comprising a wax or oil barrier component and wherein the wash assay stage is connected by a channel to a reporter bead (R-bead) assay stage (162).

3. A portable diagnostic device as claimed in claim 2, wherein said magnetic beads of the lysate stage are magnetically movable as transport beads, the reporter bead assay stage (162) contains magnetic reporter beads and the reporter beads have less magnetism than the transport beads, and the reporter beads have attached a PNA-probe ("Probe-2") complementary to target nucleic acid of the analyte, such that if the target nucleic acid is present attached to a transport bead (T-bead), a transport-bead-reporter-bead tethered sandwich is formed within the assay stage.

4. A portable diagnostic device as claimed in claim 3, wherein the sensor (168) is adapted to perform detection based on capacitance to detect the number of reporter beads on the sensor surface, in which the reporter beads represent quantity of analyte such as target nucleic acid

5. A device as claimed in any preceding claim, wherein the device comprises an agitator (176, 177) for pushing and pulling analyte over the sensor.

6. A device as claimed in any of claims 3 to 5, wherein PNA probes on the sensor are complementary to a standard PNA probe on each type of reporter (R-bead) bead, and the reporter beads are additionally functionalised with HIV_Probe2 and HCV_Probe2.

7. A device as claimed in any preceding claim, wherein the sensor comprises a trap comprising electrodes arranged perpendicular to a flow of beads and having a pitch so that only the small reporter beads can fit between them.

8. A portable diagnostic system comprising a device of any of claims 1 to 7 and a reader (200), the reader comprising:
a support (210) for receiving the device;
magnets (203, 204, 205, 206) on a drive (201, 202) to convey the magnetic beads through the assay stages of the device;
a heat source (210);
a means (210) of connection with the sensor of the device, or a sensor for coupling with the sensor interface (169) of the device, and
a controller.

9. A system as claimed in claim 8, wherein the magnets have a drive (201, 202) with a static magnet on one side and an oscillating magnet on the opposed side of a channel.

10. A diagnostic method carried out by a system of either of claims 8 or 9, the method comprising the steps of:
introducing a sample into the lysate stage and the lysis buffer lysing the sample in the lysate stage to provide an analyte, or introducing a previously-lysed analyte into the device;
conveying the analyte through the assay stages within the device wherein movement and temperature of stages are controlled by the device, and
detecting analyte at the sensor.

11. A diagnostic method as claimed in claim 10, wherein the lysis stage contains transport beads, and said beads travel out of the lysate stage and into a channel microfluidic containing molten wax in a contiguous column which has connection to aqueous reservoirs and the interfacial tension and capillary force keep assay stage reservoirs isolated from each other.

12. A diagnostic method as claimed in either of claims 10 or 11, wherein the lysis buffer is adjusted so as to only cause lysis in certain target cells or capsids in the sample, and optionally this adjustment involves a drug to bind to a particular protein target on the viral surface which damages membrane integrity.

13. A diagnostic method as claimed in any of claims 10 to 12, wherein the probe also contains cell-penetrating peptides (e.g. trans-activating transcriptional activator TAT) to facilitate cellular uptake prior to lysis, limiting exposure of the NA target to nucleases prior to lysis.

14. A diagnostic method as claimed in any of claims 10 to 13, wherein the R-beads are produced using a fluidic system to be doped with materials such as Titania or Barium Titanate to give a strong signal to a capacitance sensor.

15. A diagnostic method as claimed in any of claims 10 to 14, wherein PNA probes on the sensor are complementary to a standard PNA probe on each type of R bead.

## Patentansprüche

1. Tragbare Diagnosevorrichtung, die Folgendes umfasst:
eine Reihe von Untersuchungsplattformen (161-164), jede mit einem Speicher, der durch Kanäle (166, 171) verbunden ist;
eine Lysatplattform (165) für die Aufnahme einer Probe, wobei die Lysatplattform magnetische Kügelchen mit einer Sonde und einen Lysepuffer enthält und Folgendes umfasst:
einen Auslass zu den Untersuchungsplattformen und
ein Flüssig-Flüssig-Aufreinigungsmittel in dem Auslass, und das einen Wachsstopfen umfasst, der die magnetisch Kügelchen einschließt, die in dem Wachsstopfen für die Anbringung der Sonde an den Zielanalyten eingebettet sind, was eine Konzentration und Trennung eines Analyten von anderem Material bereitstellt, wenn er durch den Auslass hindurchgeht; und
eine Sensoruntersuchungsplattform oder eine Anschlussstelle zu einem Sensor für die Detektion von Kügelchen, die an den Analytmolekülen angebracht sind.

2. Tragbare Diagnosevorrichtung nach Anspruch 1, wobei mindestens eine Untersuchungsplattform (161, 163) zur Durchführung einer Waschung konfiguriert ist und eine Barriere (166, 171) in dem Kanal zwischen mindestens zwei Untersuchungsplattformen vorliegt, wobei die Barriere eine Wachs- oder Ölbarrierekomponente umfasst und wobei die Waschuntersuchungsplattform durch einen Kanal mit einer Untersuchungsplattform (162) mit Reporter-Kügelchen (R-Kügelchen) verbunden ist.

3. Tragbare Diagnosevorrichtung nach Anspruch 2, wobei die magnetischen Kügelchen der Lysatplattform als Transport-Kügelchen magnetisch beweglich, die Untersuchungsplattform (162) mit Reporter-Kügelchen magnetische Reporter-Kügelchen enthält und die Reporter-Kügelchen einen geringeren Magnetismus aufweisen als die Transport-Kügelchen und die Reporter-Kügelchen eine angebrachte PNA-Sonde ("Probe-2" = "Sonde-2") aufweisen, die zur Zielnukleinsäure des Analyten komplementär ist, sodass, wenn die Zielnukleinsäure an einem Transport-Kügelchen (T-Kügelchen) angebracht vorliegt, eine Transport-Kügelchen-Reporter-Kügelchenangebundene Schichtstruktur ("Sandwich") innerhalb der Untersuchungsplattform ausgebildet wird.

4. Tragbare Diagnosevorrichtung nach Anspruch 3, wobei der Sensor (168) zur Durchführung einer Detektion basierend auf Kapazität geeignet ist, um die Anzahl von Reporter-Kügelchen auf der Sensoroberfläche zu detektieren, in welcher die Reporter-Kügelchen die Menge an Analyt, wie zum Beispiel Zielnukleinsäure, repräsentieren.

5. Vorrichtung nach einem vorstehenden Anspruch, wobei die Vorrichtung eine Mischeinrichtung (176, 177) zum Schieben und Ziehen von Analyt über den Sensor umfasst.

6. Vorrichtung nach einem der Ansprüche 3 bis 5, wobei PNA-Sonden auf dem Sensor komplementär zu einer standardmäßigen PNA-Sonde auf jeder Art von Reporter-Kügelchen (R-Kügelchen) sind und die Reporter-Kügelchen zusätzlich mit HIV_Probe2 (HIV-Sonde2) und HCV_Probe2 (HCV-Sonde2) funktionalisiert sind.

7. Vorrichtung nach einem vorstehenden Anspruch, wobei der Sensor eine Falle umfasst, die Elektroden umfasst, die senkrecht zu einem Strom von Kügelchen angeordnet sind und einen Abstand aufweisen, sodass nur die kleinen Reporter-Kügelchen dazwischen passen können.

8. Tragbares Diagnosesystem, das eine Vorrichtung nach einem der Ansprüche 1 bis 7 und eine Leseeinrichtung (200) umfasst, wobei die Leseeinrichtung Folgendes umfasst:
eine Halterung (210) für die Aufnahme der Vorrichtung;
Magneten (203, 204, 205, 206) an einem Getriebe (201, 202), um die magnetischen Kügelchen durch die Untersuchungsplattformen der Vorrichtung zu befördern;
eine Wärmequelle (210);
ein Mittel (210) zur Verbindung mit dem Sensor der Vorrichtung oder einen Sensor für die Verbindung mit der Sensoranschlussstelle (169) der Vorrichtung und einen Regler.

9. System nach Anspruch 8, wobei die Magneten ein Getriebe (201, 202) mit einem statischen Magneten an einer Seite und einem oszillierenden Magneten an der gegenüberliegenden Seite eines Kanals aufweisen.

10. Diagnoseverfahren, das durch ein System nach einem der Ansprüche 8 oder 9 durchgeführt wird, wobei das Verfahren die folgenden Schritte umfasst:
Einführen einer Probe in die Lysatplattform und des Lysepuffers, der die Probe in der Lysatplattform lysiert, um einen Analyten bereitzustellen, oder Einführen eines zuvor lysierten Analyten in die Vorrichtung;
Befördern des Analyten durch die Untersuchungsplattformen innerhalb der Vorrichtung, wobei die Bewegung und Temperatur der Plattformen durch die Vorrichtung geregelt werden, und
Detektieren des Analyten an dem Sensor.

11. Diagnoseverfahren nach Anspruch 10, wobei die Lyseplattform Transport-Kügelchen enthält und sich die Kügelchen aus der Lysatplattform heraus und in einen mikrofluidischen Kanal bewegen, der geschmolzenes Wachs in einer durchgehenden Säule enthält, die mit wässrigen Speichern verbunden ist, und die Grenzflächenspannung und Kapillarkraft die Untersuchungsplattformspeicher voneinander isoliert halten.

12. Diagnoseverfahren nach einem der Ansprüche 10 oder 11, wobei der Lysepuffer derart eingestellt ist, dass nur die Lyse in bestimmten Zielzellen oder Kapsiden in der Probe bewirkt wird, und optional diese Einstellung ein Arzneimittel involviert, um ein spezielles Proteinziel auf der Virusoberfläche zu binden, das die Membranintegrität schädigt.

13. Diagnoseverfahren nach einem der Ansprüche 10 bis 12, wobei die Sonde auch in die Zelle eindringende Peptide (z. B. transaktivierenden transkriptionellen Aktivator TAT) enthält, um die zelluläre Aufnahme vor der Lyse zu unterstützen, was die Exposition des NA-Ziels gegenüber Nukleasen vor der Lyse einschränkt.

14. Diagnoseverfahren nach einem der Ansprüche 10 bis 13, wobei die R-Kügelchen unter Verwendung eines fluidischen Systems erzeugt werden, um mit Materialien, wie zum Beispiel Titandioxid oder Bariumtitanat, dotiert zu werden, um ein starkes Signal an einen Kapazitätssensor zu geben.

15. Diagnoseverfahren nach einem der Ansprüche 10 bis 14, wobei PNA-Sonden auf dem Sensor komplementär zu einer standardmäßigen PNA-Sonde auf jeder Art von R-Kügelchen sind.

## Revendications

1. Dispositif de diagnostic portatif, comprenant :
une série de stades de dosage (161-164), chacun pourvu d'un réservoir relié par des canaux (166, 171) ;
un stade de lysat (165) destiné à recevoir un échantillon, ledit stade de lysat contenant des billes magnétiques avec une sonde et un tampon de lyse, et comprenant :
une sortie vers les stades de dosage, et
un moyen de purification liquide-liquide dans la sortie et comprenant un bouchon en cire comportant lesdites billes magnétiques incluses dans le bouchon en cire pour la fixation de la probe à un analyte cible, à des fins de concentration et de séparation d'un analyte à partir d'un autre matériau au fur et à mesure de son passage à travers la sortie ; et
un stade de dosage par capteur ou une interface avec un capteur afin de détecter les billes fixées aux molécules d'analyte.

2. Dispositif de diagnostic portatif selon la revendication 1, dans lequel au moins un stade de dosage (161, 163) est configuré afin de mettre en œuvre un lavage, et il existe une barrière (166, 171) dans le canal entre au moins deux stades de dosage, ladite barrière comprenant un composant de barrière de cire ou d'huile et où le stade de dosage de lavage est relié par un canal à un stade de dosage de billes rapporteuses (billes R) (162).

3. Dispositif de diagnostic portatif selon la revendication 2, dans lequel lesdites billes magnétiques du stade de lysat sont déplaçables de manière magnétique comme billes de transport, le stade de dosage de billes rapporteuses (162) contient des billes rapporteuses magnétiques et les billes rapporteuses possèdent moins de magnétisme que les billes de transport, et les billes rapporteuses portent une sonde d'APN (« Sonde 2 ») complémentaire d'un acide nucléique cible de l'analyte, de sorte que, si l'acide nucléique cible est présent fixé à une bille de transport (bille T), un sandwich de billes de transport-billes rapporteuses assemblées est formé au sein du stade de dosage.

4. Dispositif de diagnostic portatif selon la revendication 3, dans lequel le capteur (168) est adapté afin de mettre en œuvre une détection basée sur la capacitance afin de détecter le nombre de billes rapporteuses à la surface du capteur, où les billes rapporteuses représentent la quantité d'analyte tel que l'acide nucléique cible.

5. Dispositif selon l'une quelconque des revendications précédentes, le dispositif comprenant un agitateur (176, 177) destiné à pousser et tirer l'analyte sur le capteur.

6. Dispositif selon l'une quelconque des revendications 3 à 5, dans lequel les sondes d'APN sur le capteur sont complémentaires d'une sonde d'APN standard sur chaque type de bille rapporteuse (bille R), et les billes rapporteuses sont en outre fonctionnalisées par VIH_Sonde2 et VHC_Sonde2.

7. Dispositif selon l'une quelconque des revendications précédentes, dans lequel le capteur comprend une trappe comprenant des électrodes disposées perpendiculairement à un courant de billes et ayant un pas tel que seules les petites billes rapporteuses peuvent se loger entre elles.

8. Système de diagnostic portatif comprenant un dispositif selon l'une quelconque des revendications 1 à 7 et un lecteur (200), le lecteur comprenant :
un support (210) destiné à recevoir le dispositif ;
des aimants (203, 204, 205, 206) sur un mécanisme d'entraînement (201, 202) afin de transporter les billes magnétiques à travers les stades de dosage du dispositif ;
une source de chaleur (210) ;
un moyen (210) de connexion avec le capteur du dispositif, ou un capteur destiné à un couplage avec l'interface de capteur (169) du dispositif ; et
un contrôleur.

9. Système selon la revendication 8, dans lequel les aimants possèdent un mécanisme d'entraînement (201, 202) ayant un aimant statique d'un côté et un aimant oscillant sur le côté opposé d'un canal.

10. Méthode de diagnostic mise en œuvre par un système selon la revendication 8 ou bien 9, la méthode comprenant les étapes consistant à :
introduire un échantillon dans le stade de lysat et le tampon de lyse lysant l'échantillon dans le stade de lysat afin de fournir un analyte, ou introduire un analyte préalablement lysé dans le dispositif ;
transporter l'analyte à travers les stades de dosage au sein du dispositif, où le mouvement et la température des stades sont contrôlés par le dispositif ; et
détecter l'analyte au niveau du capteur.

11. Méthode de diagnostic selon la revendication 10, dans laquelle le stade de lyse contient des billes de transport, et lesdites billes sortent du stade de lysat et rentrent dans un canal microfluidique contenant de la cire fondue dans un colonne contiguë qui est reliée à des réservoirs aqueux et la tension superficielle et la force capillaire maintiennent les réservoirs de stade de dosage isolés les uns des autres.

12. Méthode de diagnostic selon la revendication 10 ou bien 11, dans laquelle le tampon de lyse est ajusté de façon à ne provoquer la lyse que dans certaines cellules ou capsides cibles dans l'échantillon, et éventuellement cet ajustement implique la fixation d'un médicament à une cible protéique particulière à la surface virale qui endommage l'intégrité membranaire.

13. Méthode de diagnostic selon l'une quelconque des revendications 10 à 12, dans laquelle la sonde contient également des peptides de pénétration cellulaire (par exemple un activateur transcriptionnel de transactivation, TAT) afin de faciliter l'absorption cellulaire préalablement à la lyse, limitant l'exposition de la cible d'AN à des nucléases préalablement à la lyse.

14. Méthode de diagnostic selon l'une quelconque des revendications 10 à 13, dans laquelle les billes R sont produites en utilisant un système fluidique à doper par des matériaux tels que le dioxyde de titane ou le titanate de baryum afin de donner un signal important à un capteur de capacitance.

15. Méthode de diagnostic selon l'une quelconque des revendications 10 à 14, dans laquelle les sondes d'APN sur le capteur sont complémentaires d'une sonde d'APN standard sur chaque type de bille R.
